# EUROPEAN PATENT SPECIFICATION

(11) **EP 4 452 192 B1**
(45) Date of publication and mention of the grant of the patent: **23.07.2025**
(21) Application number: 22834540.1
(22) Date of filing: 08.12.2022
(51) Int. Cl.: A61K 8/26, A61K 8/28, A61K 8/39, A61K 8/86, A61K 8/92, A61Q 15/00

(54) **ANTIPERSPIRANT COMPOSITION**
SCHWEISSHEMMENDE ZUSAMMENSETZUNG
COMPOSITION ANTITRANSPIRANTE

(30) Priority: 21.12.2021 EP 21216324
(43) Date of publication of application: 30.10.2024
(73) Proprietor: Unilever IP Holdings B.V., 3013 AL Rotterdam (NL); Unilever Global IP Limited, Wirral, Merseyside CH62 4ZD (GB)
(72) Inventor: DAS, Somnath, 6708 WH Wageningen (NL); SAMADDER, Satyajit, 6708 WH Wageningen (NL)
(74) Representative: Whaley, Christopher
(86) International application number: PCT/EP2022/084892
(87) International publication number: WO 2023/117442

(56) References cited:
- US-A- 5 833 964
- DATABASE GNPD [online] MINTEL; 16 October 2019 (2019-10-16), ANONYMOUS: "24H Anti-Perspirant Stick", XP055935425, retrieved from https://www.gnpd.com/sinatra/recordpage/6947563/ Database accession no. 6947563
- DATABASE GNPD [online] MINTEL; 7 January 2019 (2019-01-07), ANONYMOUS: "48H Delicate Skin Anti-Perspirant Deodorant Stick", XP055935426, retrieved from https://www.gnpd.com/sinatra/recordpage/6246535/ Database accession no. 6246535
- DATABASE GNPD [online] MINTEL; 19 June 2014 (2014-06-19), ANONYMOUS: "48h Antiperspirant Stick", XP055935427, retrieved from https://www.gnpd.com/sinatra/recordpage/2499337/ Database accession no. 2499337
- DATABASE GNPD [online] MINTEL; 23 December 2004 (2004-12-23), ANONYMOUS: "Pro Balance Anti-Perspirant Deodorant Stick", XP055935431, retrieved from https://www.gnpd.com/sinatra/recordpage/327432/ Database accession no. 327432

## Description

### Field of the Invention

The present invention relates to antiperspirant composition, particularly to antiperspirant composition having high levels of oil.

### Background of the Invention

Antiperspirant composition generally contains antiperspirant actives to reduce perspiration on application to the surface of the body, particularly to the underarm regions of the human body viz. the axilla. Antiperspirant actives used so far are typically astringent metal salts such as aluminium or zirconium salts for example, aluminium chlorohydrates or sesquichlorohydrates. Antiperspirant actives are usually incorporated in compositions at low pH, in the range of 2 to 7. Such compositions also include natural oils which may be unsaturated for example sunflower seed oil for delivering skin care benefits. The antiperspirant composition also includes other saturated oils typically to improve the application of the antiperspirant composition onto the skin. One such composition having high levels of oil is disclosed in WO 2011/040911 A1 (Colgate-Palmolive Company) where volatile silicone is partially or fully replaced with a plant oil. The composition includes an antiperspirant active, greater than 10 wt.% plant oil and less than 40 wt.% volatile silicone oil. Mintel Database, Record ID 327432, discloses an anti-perspirant composition comprising PPG-14 butyl ether, PEG-8 distearate, Helianthus Annuus seed oil and a metal based antiperspirant active.

Antiperspirant composition including unsaturated oil is known. The unsaturated oil is included in the antiperspirant composition to provide skin care benefits. Such unsaturated oils must be present within certain amount in the composition to provides good skin care benefit, while very low levels don't give the desired skin care benefits very high levels are also not desired as such composition tend to give an oily and undesirable feel upon application on skin.

The present inventors have found that when an unsaturated oil such as sunflower seed oil is present in the antiperspirant composition to provide good skin care benefits, the fabric which comes in contact with the composition in the underarm regions tends to get stained on repeated use, this problem is further aggravated when the antiperspirant composition has a higher total oil content and further includes a metal based antiperspirant active.

It is thus an object of the present invention to provide for an antiperspirant composition comprising a metal based antiperspirant active and an unsaturated oil that minimizes the problem of staining or yellowing of fabrics on repeated use-wash-rinse-dry cycles of the fabric when worn by individuals using such antiperspirant composition.

It is yet another object of the present invention to provide an antiperspirant composition having high levels of oil content and includes an unsaturated oil and which is stable along with minimizing the problem of staining or yellowing of fabrics.

It is yet another object of the present invention to provide an antiperspirant composition with good skin care benefits having unsaturated oil and high levels of oil content and which reduces the problem of staining or yellowing of fabrics.

### Summary of the Invention

The present inventors have found that the problem of staining or yellowing of fabrics is significantly lowered when a combination of specific alkoxylated aliphatic ether and specific polyalkylene glycol diester is used in an antiperspirant composition having high total oil content and an antiperspirant active and where the weight ratio of the polyalkylene glycol diester and alkoxylated aliphatic ether in the composition ranges from 0.05 to 2. It is also found that the composition remains stable over prolonged storage.

According to a first aspect of the present invention disclosed is an antiperspirant composition comprising:
(i) an alkoxylated aliphatic ether having the general formula (IA) or (IB)

   R-O-(C₂H₄O)ₙ-H ........... (IA)

   Or,

   R-O-(C₃H₆O)ₙ-H ........... (IB);

   wherein the C₃H₆ in formulae (IB) is linear or branched, preferably branched;
   and where n is an integer from 10 to 18; and, where each R group is independently selected from a straight or a branched linear alkyl group having from 3 to 10 carbon atoms; or an aryl group or a H atom and at least one R group is chosen from the alkyl group, or the aryl group
ii) a polyalkylene glycol diester of the general formulae (IIA) or (IIB)

   R²CO.O-(C₂H₄O)ₙ-CO.R¹ ........................ (IIA)

   Or,

   R²CO.O-(C₃H₆O)ₙ-CO.R¹ ........................ (IIB)

   ); wherein the C₃H₆ in formulae (IIB) is linear or branched, preferably branched;
   and where n is an integer from 8 to 150; R² and R¹ are each independently selected from the group consisting of C₁₂ to C₁₈ alkyl group or an aryl group.
(iii) 0.1 wt.% to 5 wt.% unsaturated oil;
(iv) a metal based antiperspirant active;
wherein the total oil content including the unsaturated oil present in the composition ranges from 55 wt.% to 65 wt.% and where the weight ratio of the polyalkylene glycol diester and alkoxylated aliphatic ether in the composition ranges from 0.05 to 2.

According to a second aspect of the present invention disclosed is a method of minimizing staining or yellowish discolouration of fabric involving the steps of: applying a composition of the first aspect of the present invention to a skin of an individual, preferably the axilla (ii) washing the fabric or a portion of the fabric which comes in contact with the antiperspirant composition of the first aspect of the invention.

The composition is preferably applied on the axilla. The method is also preferably non-therapeutic or for cosmetic application.

According to a third aspect of the present invention disclosed is a use of an alkoxylated aliphatic ether, a polyalkylene glycol diester wherein the weight ratio of the polyalkylene glycol diester and alkoxylated aliphatic ether in the composition ranges from 0.05 to 2 and 0.1 wt.% to 5 wt.% unsaturated oil in antiperspirant composition with a total oil content including the unsaturated oil ranging from 55 wt.% to 65 wt.% and which composition comprises a metal based antiperspirant active for minimizing or reducing staining or yellowish discolouration of fabric which comes in contact with the composition according to the first aspect of the present invention.

By "antiperspirant composition" as used herein, is meant to include a composition for topical application to the skin of mammals, especially humans. Such a composition is preferably of the leave-on type. By a leave-on composition is meant a composition that is applied to the desired skin surface and left on for one minute to 24 hours after which it may be wiped or rinsed off with water, usually during the regular course of personal washing. The composition may also be formulated into a product which is applied to a human body for improving the appearance, cleansing, odor control or general aesthetics. The composition of the present invention can be in the form of a liquid, lotion, cream, gel or stick form and may be delivered through a roll-on device or using an aerosol can which contains a propellant. "Skin" as used herein is meant to include skin on any part of the body (e.g., neck, chest, back, arms, underarms, hands, legs, buttocks, and scalp) especially the underarms.

### Detailed Description of the Invention

Herein, all amounts, parts, percentages, and ratios are by weight and relate to the total composition, unless otherwise indicated. This also applies to the examples. Herein, the term comprising is used non-exhaustively. Herein, preferences expressed with regard to one aspect of the invention apply, to the extent possible, with regard to any other aspect of the invention. Herein, preferences expressed with regard to one feature are preferably used in combination with preferences expressed for one or more other features. Herein, when a component is referred to in the singular, it is to be understood that multiple components of the type referred to could be present and all should be included in calculating any amount or ratio specified.

Herein, cosmetic methods are to be understood as a non-therapeutic in nature and *vice versa.*

Herein, "application" and "applied" relate to application to the surface of the human body, in particular the underarm regions, unless the context dictates otherwise. The invention typically involves the topical application of the antiperspirant active to the underarm regions of the human body, otherwise known as the axillae.

According to a first aspect of the present invention disclosed is an antiperspirant composition having an alkoxylated aliphatic ether of Formula (IA or IB), a polyalkylene glycol diester of Formula (II); 0.1 wt.% to 5 wt.% unsaturated oil and a metal based antiperspirant active.

### Alkoxylated aliphatic ether

According to a first aspect of the present invention disclosed antiperspirant composition has an alkoxylated aliphatic ether. The alkoxylated aliphatic ether has an alkyl chain length of C₃ to C₁₀. The alkoxylated aliphatic ether is preferably derived from at least one alcohol having C₃ to C₁₀ carbon atoms.

The alkoxylated aliphatic ether has the general formulae (IA or IB)

R-O-(C₂H₄O)ₙ-H ........... (IA)

Or,

R-O-(C₃H₆O)ₙ-H ........... (IB);

wherein the C₃H₆ in formulae (IB) is linear or branched, preferably branched;
and where n is an integer from 10 to 18, preferably 12 to 16 and more preferably 14; where each R group is independently selected from a straight or a branched linear alkyl group having from 3 to 10 carbon atoms, preferably from 3 to 5 carbon atoms and more preferably 4 carbon atoms, or an aryl group or a H atom and at least one R group is chosen from an alkyl group or an aryl group. More preferably at least one R group is a linear alkyl group. Most preferably the linear alkyl chain is butyl or t-butyl.

Preferably the alkoxylated aliphatic ether is R-O-(C₃H₆O)ₙ-H wherein the C₃H₆ is linear or branched, preferably branched. More preferably, the alkoxylated aliphatic ether is polypropylene glycol 14 ether of butyl alcohol. A suitable example is obtainable under the CTFA INCI approved name of PPG-14-butyl ether. Commercially available alkoxylated aliphatic ether are available from Dow under the trade name Fluid AP.

The composition according to the present invention includes from 1 wt.% to 25 wt.% alkoxylated aliphatic ether. Preferably the composition includes from 2 wt.% to 25 wt.%, still preferably from 5 wt.% to 25 wt.% alkoxylated aliphatic ether of Formula (IA) or Formula (IB). Preferably the composition includes from 10 wt.% to 25 wt.% alkoxylated aliphatic ether, more preferably the composition has not less than 6 wt.%, still more preferably less than 8 wt.%, still more preferably not less than 10 wt.% alkoxylated aliphatic ether but preferably the amount of the alkoxylated aliphatic ether in the composition is not more than 25 wt.%, still preferably not more than 20 wt.%.

### Polyalkylene glycol diester

According to a first aspect of the present invention disclosed antiperspirant composition has a polyalkylene glycol diester. The polyalkylene glycol diester has a linear alkyl group with a chain length of C₁₂ to C₁₈. The polyalkylene glycol diester is preferably derived from at least one fatty alcohol having C₁₂ to C₁₈ carbon atoms. The polyalkylene glycol diester has polyalkylene oxide unit per mole ranging from 8 to 150, preferably the polyalkylene oxide is polyethylene oxide (EO).

The polyalkylene glycol diester has the general formulae (IIA) or (IIB)

R²CO.O-(C₂H₄O)ₙ-CO.R¹ ........................ (IIA)

Or,

R²CO.O-(C₃H₆O)ₙ-CO.R¹ ........................ (IIB)

); wherein the C₃H₆ in formulae (IB) is linear or branched, preferably branched;
and where n is an integer from 8 to 150, preferably 8 to 22 and more preferably 8.; R² and R¹ are each independently selected from the group consisting of C₁₂ to C₁₈ alkyl group or an aryl group. Preferably the R² and R¹ are each independently selected from the group consisting of C₁₂ to C₁₈ alkyl group, more preferably C₁₆ alkyl group.

Most preferably R² and R¹ are same. Preferably, the polyalkylene glycol diester is R²CO.O-(C₂H₄O)ₙ-CO.R¹ where n is preferably from 8 to 150, still more preferably n is 8 to 22, most preferably n is 8.

Suitable examples of the polyalkylene glycol diester having 8 to 150 polyalkylene oxide units (preferably polyethylene oxide EO units) per mole of the diester is selected from the group consisting of PEG-dilaurates, PEG dioleates, PEG-distearates, PEG-isostearates, PEG-laurates, PEG-dilinolenates, PEG dimyristates, PEG-dioleates, PEG-dipalmitates, PEG-diricinoleates, or mixtures thereof. A suitable example is obtainable under the CTFA INCI approved name of PEG-8-Distearate. Commercially available PEG-8 Distearate are available from Croda under the trade name Cithrol^{™} 4DS.

The composition according to the present invention includes from 0.1 wt.% to 5 wt.% polyalkylene glycol diester. Preferably the composition includes from 0.1 wt.% to 3 wt.% polyalkylene glycol diester, still preferably from 0.1 to 2 wt.%, but preferably the composition has not less than 0.7 wt.%, still more preferably less than 0.8 wt.%, still more preferably not less than 1 wt.% polyalkylene glycol diester but preferably the amount of the polyalkylene glycol diester is not more than 4.5 wt.%, still preferably not more than 3.5 wt.% in the antiperspirant composition.

Preferably the weight ratio of polyethylene glycol diester to alkoxylated aliphatic ether in the composition is in the range from 0.05 to 2, still preferably from 0.09 to 2, still more preferably from 1 to 2. The composition according to the present invention thus has higher levels of alkoxylated aliphatic ether as compared to polyalkylene glycol diester by weight of the composition. Preferably the polyethylene glycol diester is PEG-8 distearate and alkoxylated aliphatic ether PPG 14 Butyl ether. It is preferred that the weight ratio of polyethylene glycol diester to alkoxylated aliphatic ether (preferably weight ratio between PEG-8 Distearate and PPG 14 Butyl ether) in the composition is not more than 2, at weight ratio above 2 the composition becomes slippery (negative sensory) and has reduced consumer acceptability. Moreover, higher levels of polyethylene glycol diester (preferably PEG-8 Distearate) in the composition negatively impacts the respiratory system in non-contact formats and may lead to skin irritation. Specifically in a stick form, the weight ratio of polyethylene glycol diester to alkoxylated aliphatic ether above 2 severely impacts the mechanical integrity of the stick.

### Unsaturated oil

According to a first aspect of the present invention disclosed antiperspirant composition includes an unsaturated oil.

The amount of unsaturated oil in the range from 0.1 wt.% to 5 wt.% is added in the antiperspirant composition to provide good skin care benefits. A lower level of at least 0.1 wt.% is required to provide good skin care benefits. While higher level of the unsaturated oil in the composition may provide good skin care benefits, but at an amount above 5 wt.% the composition tends to become oily on application and is not acceptable to the consumer. More preferably the unsaturated oil is present in an amount ranging from 0.3 wt.% to 5 wt.% also preferred are ranges from 1 wt. % to 5 wt. % or from 2 wt. % to 4 wt.%.

Preferably the unsaturated oil is advantageously a plant oil and particularly is a triglyceride oil. Such oils are often obtainable by extraction from the plant seed. Suitable plant oils include sunflower seed oil, maize corn oil, evening primrose oil, coriander seed oil, safflower oil, olive oil, rape seed oil, castor oil and borage seed oil. Most preferably the unsaturated oil is a sunflower seed oil. It is particularly desirable to employ an unsaturated oil which comprises mono or polyunsaturated long chain aliphatic carboxylate substituents, such as notably C₁₈ carboxylates containing 1, 2 or 3 degrees of unsaturation, 2 or more of which may be conjugated.

Preferably the unsaturated oil has an iodine value of at least 100. Still preferably the iodine value ranges from 100 to 150. Iodine value is a measure of the amount of unsaturation in the oil, expressed in the mass of iodine in grams that is consumed by 100 grams of the oil. A low iodine value implies a high level of saturation, and vice-versa. The iodine value can be determined by the WIJ'S method of the American Oil Chemists Society (A.O.C.S. Cd1-25).

The antiperspirant composition according to the first aspect of the present invention comprises from 0.1 wt.% 5 wt.% unsaturated oil. Preferably the antiperspirant composition comprises at least 0.2 wt.%, still preferably at least 0.3 wt.%, still preferably at least 0.4 wt.%, most preferably at least 0.5 wt.% of the unsaturated oil, but typically not more than 5 wt.%, still preferably not more than 4.5 wt.%, still further preferably not more 4 wt.% and most preferably not more than 2 wt.% of unsaturated oil based on the weight of the antiperspirant composition.

### Oily Emollient

According to a first aspect of the present invention disclosed antiperspirant composition preferably includes an oily emollient. The term "oily emollient" as used herein refers to a lipid material which is immiscible in water at room temperature (25°C) and pressure (1atm), and which is in the liquid state at room temperature and pressure, and which preferably has a specific gravity lower than water at standard temperature and pressure conditions (25°C and 1 atmospheric pressure).

Preferably the oily emollient according to the present invention includes those selected from the group consisting of cyclomethicone, benzoate ester, non-volatile silicone oil preferably dimethicones and mixtures thereof.

The oily emollient may be preferably cyclomethicone. As used herein the term "cyclomethicone" refers to the group of methyl siloxane, cyclic polydimethylsiloxane polymer which are commonly used as skin emollients in antiperspirant composition. Suitable examples of the cyclomethicone includes hexamethylcyclotrisiloxane (CAS: 541-05-9), octamethylcyclotetrasiloxane (CAS: 556-67-2), decamethylclopentasiloxane (CAS: 541-02-6), and dodecamethylcyclohexasiloxane (CAS: 540-97-6), cyclopentasiloxane or mixtures thereof. Cyclopentasiloxane is particularly preferred. Commercially available cyclomethicone include but not limited to grade designations 344, 345, 244, DC245 and 246 from Dow Corning Corporation; Silicone 7207(TM) and Silicone 7158(TM) from Union Carbide Corporation; and SF1202(TM) from General Electric.

Preferably the amount of the cyclomethicone oily emollient in the composition ranged from 0 wt.% to 50 wt.%, still preferably 5 wt.% to 35 wt.% and most preferably from 10 wt.% to 30 wt.% of the antiperspirant composition according to the present invention. The present invention also include compositions with low silicone oil levels. By low silicone oil levels is meant composition having 0 wt.% to 25 wt.%, still preferably from 0 wt.% to 20 wt.%, more preferably from 0 wt.% to 15 wt.%, still more preferably 0 wt.% to 10 wt.% and furthermore preferably from 0 wt.% to 5 wt.% or less silicone oil.

The oily emollient may be preferably a benzoate ester. Preferably an alkyl benzoate, more preferably C₈ to C₁₈ alkyl benzoates, still preferably C₁₂ to C₁₅ alkyl benzoates. Many suitable benzoate esters are available under the trademark Finsolv^{™} from Innospec. Preferably the amount of the benzoate ester oily emollient in the composition ranged from 0 wt.% to 20 wt.%, still preferably 5 wt.% to 15 wt.% and most preferably from 10 wt.% to 10 wt.% of the antiperspirant composition according to the present invention.

The oily emollient may be preferably a non-volatile silicone oil selected from the group consisting of dimethicones, linear alkylarylsiloxanes and mixtures thereof. Particularly suitable polysiloxanes include polydimethylsiloxanes, which are also known as dimethicones, with viscosities of between 10 cst and 1000 cst, (e.g., between 15 cst to 400 cst or even between 20 cst and 200 cst). The average chain length for these preferred dimethicone materials is from 12 to 375 dimethylsiloxane units (e.g., from 20 to 200 or even from 27 to 125). The dimethicone typically have an intermediate chain length, such as from 20 to 100 silicon atoms. The alkylarylsiloxanes are particularly those containing from 2 to 4 silicon atoms and at least one phenyl substituent per silicon atom, or at least one diphenylene group. Commercially available non-volatile silicone oil includes but not limited to XIAMETER^{™} PMX-200 Silicone Fluid 50 cSt from Dow. Preferably the amount of the non-volatile silicone oil oily emollient in the composition ranged from 0 wt.% to 5 wt.%, still preferably 0.5 wt.% to 3 wt.% and most preferably from 1 wt.% to 3 wt.% of the antiperspirant composition according to the present invention.

According to the present invention, the term "total oil content" refers to the sum total of the oily component in the composition. The oily component is defined as a lipid material which is immiscible in water at room temperature (25°C) and pressure (1atm), and which is in the liquid state at room temperature and pressure, and which preferably has a specific gravity lower than water at standard temperature and pressure conditions (25°C and 1 atmospheric pressure). In accordance with the present invention the total oil content includes the unsaturated oil, the oily emollient and the alkoxylated aliphatic ether as defined in the present invention.

The sum total of the amount of the unsaturated oil, the alkoxylated aliphatic ether and the amount of the preferred oily emollient present in the composition based on the weight of the antiperspirant composition ranges from 55 wt.% to 65 wt.%, still preferably from 55 wt.% to 63 wt.%. Preferably the antiperspirant composition comprises at least 56 wt.%, still preferably at least 58 wt.%, still preferably at least 59 wt.%, most preferably at least 60 wt.% of the total oil content, but typically not more than 64.5 wt.%, still preferably not more than 64 wt.%, still further preferably not more and most preferably not more than 63 wt.% of total oil content based on the weight of the antiperspirant composition.

When the antiperspirant composition is an aerosol composition, the total oil content is calculated based on the total weight of the base composition. Where the base composition is defined as the weight of the aerosol antiperspirant composition devoid of the propellant. The total oil content is preferably calculated as the sum total of the amount of the unsaturated oil, alkoxylated aliphatic ether and the amount of the preferred oily emollient.

The weight of fragrance materials is not included herein in calculating the weight of the oil content, irrespective of whether the fragrance is encapsulated or "free".

### A metal based antiperspirant active

According to a first aspect of the present invention disclosed antiperspirant composition includes a metal based antiperspirant active.

The metal based antiperspirant active may be selected from an aluminium, zirconium or mixed aluminium/zirconium salts, preferably, aluminium chlorohydrate, aluminum-zirconium tetrachlorohydrex glycine complex, aluminum-zirconium octachlorohydrex glycine complex, aluminum-zirconium pentachlorohydrate, aluminum sesquichlorohydrate or mixtures thereof. Antiperspirant actives for use herein are selected from aluminium, zirconium and mixed aluminium/zirconium salts, including both inorganic salts, salts with organic anions and complexes. Particularly preferred astringent salts are halohydrate salts, and especially chlorohydrate salts, optionally activated. For aerosol compositions, the antiperspirant active is preferably free from zirconium.

Aluminium halohydrates are usually defined by the general formula Alₓ(OH)_{y}Q_{z}.nH₂O where 1/0<x/y<1/3; y+z=3. Preferably x, y, z are integers or fractions. Q represents a halogen selected from the group consisting of chlorine, bromine, iodine, or sulphate or mixtures thereof and nH₂O represents a variable amount of hydration. Especially effective aluminium halohydrate salts, known as activated aluminium chlorohydrates, are described in EP-A-6739 (Unilever NV et al).

The term aluminium chlorohydrate herein encompasses materials with specified figures for y and z, such as aluminium sesquichlorohydrate and materials in which the chlorohydrate is present as a complex. It will be recognized that alternative names are sometimes used to indicate the presence of hydroxyl substitution, including aluminium hydroxy chloride, aluminium oxychloride or basic aluminium chloride.

Zirconium astringent salts for employment herein as the metal based antiperspirant active can usually be represented by the empirical general formula: ZrO(OH)_{2n-nz}B_{z}.wH₂O in which z is a variable in the range of from 0.9 to 2.0 so that the value 2n-nz is zero or positive, n is the valency of B, and B is selected from the group consisting of chloride, other halide, sulphamate, sulphate or mixtures thereof. Possible hydration to a variable extent is represented by wH₂O. Preferably, B represents chloride. Preferably, the variable z lies in the range from 1.5 to 1.87. In practice, such zirconium salts are commonly not employed by themselves, but as a component of a combined aluminium and zirconium-based antiperspirant active.

The above aluminium and zirconium salts may have coordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed as metal based antiperspirant active. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula CH₂(NH₂)COOH. Certain of those Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine. Actives of this preferred type are available from Westwood, from Summit and from Reheis. Alternatively, the complex can be pre-formed with a polyhydric aliphatic alcohol, such as propylene glycol or glycerol. A complex with a chlorohydrate is commonly referred to as a chlorhydrex.

Mixtures of two or more astringent salts can be employed, but, however, it is particularly preferred to employ astringent salts that are free from zirconium, such as aluminium chlorohydrates and so-called activated aluminium chlorohydrates.

According to an especially preferred aspect of the present invention the antiperspirant active is aluminium chlorohydrate, aluminum sesquichlorohydrate or mixtures thereof. The metal based antiperspirant active preferably is included in 1 wt.% to 50 wt.%, more preferably 2 wt.% to 25 wt.%, most preferably 2 wt.% to 15 wt.% by weight of the composition.

### Additional preferred ingredients

### Aliphatic alcohol:

Water- immiscible aliphatic alcohols can be beneficially employed, particularly those having a boiling point of higher than 100°C. These include fatty alcohol having at least 10 carbon atoms and, in many instances, up to 30 carbon atoms, particularly 15 to 25. The alcohol is linear and preferably has one fatty alcohol or a blend of fatty alcohol may be employed. Suitable examples include cetyl alcohol, stearyl alcohol, eicosyl alcohol, behenyl alcohol, isostearyl alcohol, hexyldecanol and octyl-dodecanol. Such alcohols can often constitute from 5 to 25 wt.%, still preferably 5 wt.% 20 wt.% of the composition. Most preferably the alcohol is stearyl alcohol. The alcohol is very desirably stearyl alcohol, including commercially available material containing up to about 10% by weight of alcohols containing 16, 20 and/or 22 carbons. Non-limiting example of commercially available aliphatic alcohol includes Lanette C18 Deo from Croda.

### Polyethylene:

Preferably the antiperspirant composition according to the present invention may include polyethylene. Preferably the polyethylene having a melting point of 75°C and preferably >78°C. Particularly desirably, the polyethylene has a melting point of up to 85°C. Suitable polyethylene often has an average molecular weight in the region of 400 to 540 Daltons, such as from 420 to 500 Daltons. An especially preferred example is Performalene 400 which has a molecular weight of about 450 to 460. Preferably the polyethylene is present in an amount of from 0.5 to 3.5% by weight of the composition.

### Ester wax:

Preferably the antiperspirant composition according to the present invention may include an ester wax. The ester wax can be a monoester or a multi-ester. The ester wax can be fractionated from a natural wax such as from beeswax. Or be a synthetic analogue. A particularly suitable wax comprises a hydrogenated plant ester oil such a castor oil, the extent of hydrogenation of the unsaturated C₁₈ substituent being sufficient to attain the desired melting point. It will be recognized that in order to satisfy the instant invention, the castor wax must be one selected to attain the desired melting point criterion. A particularly preferred ester wax is Castorwax MP80, but castor oil that has been hydrogenated to an even greater extent can be contemplated too. Alternately hydrogenated oil selected from hydrogenated soybean oil, hydrogenated palm oil, hydrogenated corn oil, hydrogenated peanut oil, hydrogenated cottonseed oil, hydrogenated olive oil, hydrogenated avocado oil, hydrogenated castor oil, or a mixture thereof may also be suitable for the present invention. Such ester wax can often constitute from 1 to 15 wt.%, still preferably 2 wt.% 10 wt.% of the composition.

### Preservative:

A preservative is a preferred additional component in compositions of the invention. A preservative serves to reduce or eliminate microbial contamination of compositions of the invention. Suitable preservatives for use with the present invention include 2-phenoxyethanol, iodopropynyl butylcarbamate, C₁-C₃ alkyl parabens, sodium benzoate, caprylyl glycol and EDTA. Particularly preferred preservatives are 2-phenoxyethanol, iodopropynyl butylcarbamate, sodium benzoate, caprylyl glycol and EDTA and especially preferred are 2-phenoxyethanol and iodopropynyl butylcarbamate. Preservatives are typically employed at a total level of from 0.05 wt.% to 3 wt.%, preferably at from 0.1 wt.% to 2 wt.% and most preferably at from 0.4 wt.% to 1 wt.% in the antiperspirant composition.

### Antimicrobial deodorant active:

An antimicrobial deodorant active is a preferred additional component in compositions of the invention. Such components serve to reduce or eliminate body odour by reducing or otherwise impeding the function of microbes on the skin of the body responsible for malodour generation. The antimicrobial deodorant active may also be a preservative for the composition. When employed, the anti-microbial deodorant agent is typically incorporated into the composition at from 0.01 wt.% to 3 wt.% and particularly at from 0.03 wt.% to 0.5 wt.%.

Preferred anti-microbial deodorant agents have a minimum inhibitory concentration (MIC) of 1 mg.ml⁻¹ or less, particularly 200 µg.ml⁻¹ or less, and especially 100 µg.ml⁻¹ or less. The MIC of an anti-microbial agent is the minimum concentration of the agent required to significantly inhibit microbial growth. Inhibition is considered "significant" if an 80% or greater reduction in the growth of an inoculum of *Staphylococcus epidermidis* is observed, relative to a control medium without an anti-microbial agent, over a period of 16 to 24 hours at 37°C. Details of suitable methods for determining MICs can be found in "Antimicrobial Agents and Susceptibility Testing", C. Thornsberry, (in "Manual of Clinical Microbiology", 5th Edition, Ed. A. Balows et al, American Society for Microbiology, Washington D.C., 1991). A particularly suitable method is the Macrobroth Dilution Method as described in Chapter 110 of above publication (pp. 1101-1111) by D. F. Sahm and J. A. Washington II. MICs of anti-microbials suitable for inclusion in the compositions of the invention are triclosan: 0.01-10 µg.ml⁻¹ (J. Regos et al., Dermatologica (1979), 158: 72-79) and farnesol: ca. 25 µg.ml⁻¹ (K. Sawano, T. Sato, and R. Hattori, Proceedings of the 17th IFSCC International Conference, Yokahama (1992) p.210-232). By contrast ethanol and similar alkanols have MICs of greater than 1 mg.ml⁻¹.

Suitable organic anti-microbials are bactericides, for example quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A. Makin and M.R. Lowry, in "Antiperspirants and Deodorants", Ed. K. Laden (1999, Marcel Dekker, New York). More preferred anti-microbials for use in the compositions of the invention are polyhexamethylene biguanide salts (also known as polyaminopropyl biguanide salts), an example being Cosmocil CQ^{™} available from Zeneca PLC, preferably used at up to 1% and more preferably at 0.03% to 0.3% by weight; 2',4,4'-trichloro,2-hydroxy-diphenyl ether (triclosan), preferably used at up to 1% by weight of the composition and more preferably at 0.05 to 0.3%; and 3,7,11-trimethyldodeca-2,6,10-trienol (farnesol), preferably used at up to 1% by weight of the composition and more preferably at up to 0.5%.

Other suitable organic antimicrobial agents are transition metal chelators, as described in WO01/52805, for example. Transitional metal chelators having a binding coefficient for iron (III) of greater than 10²⁶, for example diethylenetriaminepentaacetic acid and salts thereof are preferred.

### Antioxidants:

Antioxidants may be advantageously employed in the compositions. Antioxidants that may be used include butylated hydroxytoluene (BHT), butylated hydroxyanisole (BHA) and pentaerythrityl tetra-di-t-butyl hydroxyhydrocinnamate. Antioxidants may be used in an amount ranging from 0.05 wt.% to 5 wt.%, preferably from 0.075 wt.% to 2.5 wt.% and especially preferably in an amount ranging from 0.1 wt.% to 1 wt.%.

### Sensory modifiers:

Certain sensory modifiers are further desirable components. Such materials are preferably used at a level of up to 20 wt.% by weight of the composition. Humectants, volatile oils, non-volatile oils, and particulate solids which impart lubricity are all suitable classes of sensory modifiers. Examples of such materials include isopropyl myristate, isopropyl palmitate, talc, finely divided silica (e.g., Aerosil 200), particulate polyethylene (e.g., Acumist B18), polysaccharides, corn starch, C₁₂ to C₁₅ alcohol benzoate, octyl dodecanol, C₇ to C₁₄ isoparaffins, di-isopropyl adipate, isosorbide laurate, glycerol, hydrogenated polyisobutene, polydecene, titanium dioxide, phenyl trimethicone, dioctyl adipate, and hexamethyl disiloxane.

### Fragrance:

Fragrance is also a desirable additional component. Suitable materials include conventional perfumes, such as perfume oils and also include so-called deo-perfumes, as described in EP 545,556 and other publications. Levels of incorporation are preferably up to 4 wt.%, particularly from 0.1 wt.% to 2 wt.%, and especially from 0.7 wt.% to 1.7 wt.%.

Other components commonly included in conventional antiperspirant compositions may also be incorporated in the composition of the present invention. Such components include skin care agents such as emollients, humectants and skin barrier promoters; skin appearance modifiers such as skin brightening agents and skin smoothing agents and mixtures thereof.

### Thickening agents:

Thickening agents may be optionally employed in compositions of the invention. Such agents increase the viscosity of or solidify the carrier fluid in which the antiperspirant agent is typically suspended or dissolved.

Thickening agents may be selected from any of those known in the art, provided reasonable skill is used to avoid any incompatibilities. A preferred class of thickeners, especially for compositions also comprising water, are hydroxyalkyl celluloses, such as hydroxypropyl cellulose. When employed, thickening agents are typically used at a level of from 0.1 wt.% to 40 wt.%. When a hydroxyalkyl cellulose thickening agent is employed, this is typically used at a level of from 0.2 wt.% to 10% wt.%.

In some embodiments, the present invention may involve a stick or soft solid composition. In such compositions, a thickener is an essential additional component and is often described as a gelling agent or structurant.

The thickening agents used in stick compositions according to the invention are preferably selected from fibre-forming non-polymeric gelling agents and waxes, optionally supplemented by particulate silica and/or an oil-soluble polymeric thickening agent.

When employed, the thickening agent often comprises a wax. Thickening waxes typically melt at above 40°C and particularly at between 55°C and 95°C. Waxes can include ester waxes, including C₁₂ to C₂₄ linear fatty alcohols, waxes obtained from animals or plants, often after hydrogenation, silicone elastomers and silicone waxes. The thickening agent can comprise a mixture of particulate thickening agents, a mixture of waxes or a mixture of both types of material.

Waxes employed herein as thickening agents are often selected from hydrocarbons, linear fatty alcohols, silicone polymers, esters of fatty acids or mixtures containing such compounds along with a minority (less than 50% w/w and often less than 20% w/w) of other compounds. Naturally occurring waxes are often mixtures of compounds which include a substantial proportion of fatty esters.

Examples of hydrocarbon waxes include paraffin wax, ozakerite, microcrystalline wax and polyethylene wax, the last named desirably having an average molecular weight of from 300 to 600 and advantageously from 350 to 525.

Examples of linear fatty alcohols include those containing from 14 to 40 carbon atoms and often from 16 to 24. Preferred thickening agents of this class are stearyl alcohol and behenyl alcohol, with stearyl alcohol being especially preferred.

Examples of ester waxes include esters of C₁₆-C₂₂ fatty acids with glycerol or ethylene glycol.

Examples of natural waxes include beeswax, wool wax and spermaceti wax of animal origin, and caster wax, jojoba wax, carnauba wax and candelilla wax which are of vegetable origin.

Further waxes employable herein are silicone polymer waxes.

Fibre-forming thickening agents are dissolved in the carrier oil at elevated temperature and on cooling precipitate out to form a network of very thin strands that thicken the carrier oil. Such fibre-forming thickeners include N-acyl amino-acid amides and in particular linear and branched N-acyl glutamic acid dialkylamides, such as in particular N-lauroyl glutamic acid di n-butylamide and N-ethylhexanoyl glutamic acid di n-butylamide and especially mixtures thereof. Such amido gellants can be employed in anhydrous compositions according to the present invention, if desired, with 12-hydroxystearic acid.

When employed, the thickening agent is typically used at a concentration of from 1.5 wt. to 30 wt.%. When a fibre-forming thickening agent is employed, its concentration is typically in the range of from 1.5 wt.% to 15 wt.%. When a wax is employed, its concentration is usually selected in the range of from 10 wt.%. to 30 wt.%., and particularly from 12% to 24% w/w.

### Propellant:

In some embodiments, the present invention may involve an aerosol composition. In such compositions, a volatile propellant is preferred additional component. Preferred volatile propellants are liquefied gases, for example hydrocarbons or halogenated hydrocarbon gases (particularly fluorinated hydrocarbons such as 1,1-difluoroethane and/or 1-trifluoro-2-fluoroethane) that have a boiling point of below 10°C and especially those with a boiling point below 0°C. It is especially preferred to employ liquefied hydrocarbon gases, and especially C₃ to C₆ hydrocarbons, including propane, butane, isobutane, pentane and isopentane and mixtures of two or more thereof. Of these especially preferred propellants, isobutane, isobutane/propane, butane/propane and mixtures of propane, isobutane and butane are most preferred. Other propellants that can be contemplated include alkyl ethers, such as dimethyl ether or compressed non-reactive gasses such air, nitrogen, or carbon dioxide.

The liquefied propellant gas is typically the major component of aerosol compositions used in conjunction with the invention, often comprising from 30 wt.% to 99 wt.% and preferably comprising from 50 wt.% to 95 wt.% of the total composition.

### Antiperspirant Composition

The composition of the invention is preferably delivered as delivered as a liquid, lotion, cream, foam, scrub, gel or stick form and may be delivered through a roll-on device or using a propellant containing aerosol can. More preferably as a product in aerosol form or in stick form.

The antiperspirant composition can be applied cosmetically and topically to the skin, by a contact method which involves wiping a composition across the surface of the skin and depositing a fraction of the composition onto the skin. There are broadly speaking two classes of contact compositions. The first class of contact composition is a liquid and usually applied using a roll-on dispenser or possibly absorbed into or onto a wipe, and in the second of which the antiperspirant active is distributed within a carrier liquid that forms a continuous phase that has been gelled. In one variation, the carrier fluid comprises a solvent for the antiperspirant and in a second variation, the antiperspirant remains a particulate solid that is suspended in an oil, usually a blend of oils.

Preferably the antiperspirant composition is an anhydrous composition, more preferably the antiperspirant composition is an anhydrous composition having less than 10 wt.% water.

### Stick or soft solid compositions:

Many different materials have been proposed as gellant for a continuous oil phase, including waxes, small molecule gelling agents and polymers. They each have their advantages and of them, one of the most popular class of gellant has comprised waxes, partly at least due to their ready availability and ease of processing, including in particular linear fatty alcohol wax gellants. A gelled antiperspirant composition is applied topically to skin by wiping it across and in contact with the skin, thereby depositing on the skin a thin film.

Dispensers suitable for use with the present invention comprise containment means, and application means for the composition. They also typically comprise means for getting the composition from its containment means to its application means. In embodiments where the composition is in a solid or soft solid form, the containment means may be a stick barrel and the application means propels a composition comprising the polypeptide clear of the stick barrel and allows it to be rubbed onto skin surface.

### Roll-on:

Liquid compositions that are applicable from a roll-on broadly speaking can be divided into two classes, namely those in which an antiperspirant active is suspended in a hydrophobic carrier, such as a volatile silicone and those in which the antiperspirant active is dissolved in a carrier liquid. The latter has proven to be more popular. There are mainly two sorts of dissolving carrier liquid, namely carriers that are predominantly alcoholic, which is to say the greater part of the dissolving carrier fluid comprises ethanol and the second class in which the carrier liquid is mainly water. Since the present invention involves a hydrophobic composition, ethanol is preferred. The former is very popular because ethanol is a mild bactericide in its own right.

In embodiments where the composition is in a liquid form, the containment means is a reservoir for the composition and the application means is preferably a surface from which the composition may be applied to the skin.

In a preferred embodiment, the composition of the invention is stored in a roll-on dispenser and is dispensed therefrom. Compositions of the invention are particularly suited to being stored and dispensed from a roll-on dispenser designed to be kept in an inverted position for extended periods. For the avoidance of doubt, "an inverted position" for a roll-on dispenser is with the roll-on applicator, usually a ball, located below the reservoir containing the composition.

### Aerosol compositions:

The antiperspirant composition can be applied cosmetically and topically to the skin, by a second method, sometimes called the non-contact method. In this, the composition is sprayed from a dispenser held proximate to the skin, often in an area of about 10 cm² to 20 cm². The spray can be developed by mechanical means of generating pressure on the contents of the dispenser, such as a pump or a squeezable sidewall or by internally generated pressure arising from a fraction of a liquefied propellant volatilizing, the dispenser commonly being called an aerosol.

When the antiperspirant composition is sprayed onto the skin surface, the containment means is typically a pressurized canister and the application means is a spray nozzle through which the composition is released under pressure on release of a valve.

The antiperspirant composition may be delivered through an aerosol composition which comprises a propellant in addition to the other ingredients described hereinabove. Commonly, the propellant is employed in a weight ratio to the base formulation of from 95:5 to 5:95. Depending on the propellant, in such aerosol compositions the ratio of propellant to base formulation is normally at least 20:80, generally at least 30:70, particularly at least 40:60, and in many formulations, the weight ratio is from 90:10 to 50:50. A ratio range of from 70:30 to 90:10 is sometimes preferred.

Propellants herein generally are one of three classes; i) low boiling point gasses liquefied by compression, ii) volatile ethers and iii) compressed non-oxidizing gases.

Class i) is conveniently a low boiling point material, typically boiling below -5°C, and often below -15°C, and in particular, alkanes and/or halogenated hydrocarbons. This class of propellant is usually liquefied at the pressure in the aerosol canister and evaporates to generate the pressure to expel the composition out of the canister. Examples of suitable alkanes include particularly propane, butane or isobutane. The second class of propellant comprises a very volatile ether of which the most widely employed ether hitherto is dimethyl ether. This propellant can advantageously be employed at relatively low weight ratio of propellant to base formulation, for example to as low as 5:95. It can also be employed in admixture with, for example, compressible/liquefiable alkane gasses. The third class of propellant comprises compressed non-oxidising gasses, and in particular carbon dioxide or nitrogen. Inert gases like neon are a theoretical alternative.

The composition of the present invention can comprise a wide range of other optional components. The CTFA Personal care Ingredient Handbook, Second Edition, 1992, describes a wide variety of non-limiting personal care and pharmaceutical ingredients commonly used in the skin care industry, which are suitable for use in the compositions of the present invention. Examples include binders, biological additives, buffering agents, colorants, thickeners, polymers, astringents, fragrance, conditioners, exfoliating agents, pH adjusters, preservatives, natural extracts, essential oils, skin sensates, skin soothing agents, and skin healing agents.

The composition of the invention preferably comprises a topically acceptable carrier which in the present invention is anhydrous. To enable this, the anhydrous carrier preferably comprises a silicone compound, an alcohol or a wax. The alcohol, when used, could be a low boiling (C₂ to C₄) alcohol or a polyhydric alcohol, preferably a polyhydric alcohol.

### Method of minimizing staining

According to another aspect of the present invention there is provided a method of minimizing staining or yellowish colouration of fabric comprising the steps of (i) applying an antiperspirant composition according to the first aspect of the present invention onto the skin, preferably the axilla of an individual followed by (ii) washing the fabric in an aqueous detergent composition.

In the first step the antiperspirant composition is applied to the skin, which skin surface is preferably in contact with a fabric worn by an individual. More preferably the skin surface is the axilla. In the next step, the washing of the fabric is preferably carried in an aqueous detergent composition. The surfactant is preferably an anionic surfactant, non-limiting examples includes linear alkyl benzene sulphonate. Preferably the step of washing is followed by rinsing the laundered fabric with water. This rinsing step is preferably followed by drying the fabric.

According to a third aspect of the present invention disclosed is the use of an alkoxylated aliphatic ether with formula (I), polyalkylene glycol diester of formula (II), 0.1 wt.% to 5 wt.% unsaturated oil, in an antiperspirant composition including a metal based antiperspirant active and a total oil content including the unsaturated oil ranging from 55 wt.% to 65 wt.% for minimizing the staining or yellowing of fabric which come in contact with the composition.

The invention will now be illustrated with the help of the following non-limiting examples.

### Examples

Example 1: Anti-perspirant stick composition as shown in Table 1 below were prepared in a batch size of 500 to 1000 grams. Initially all the ingredients according to the table 1 were weighed.

To prepare the antiperspirant composition, a beaker was placed in a water bath maintained at a temperature of 85°C. The stearyl alcohol, hydrogenated castor oil and the PEG distearate was added to the beaker and melted. The melted ingredients were then added to a Silverson mixer and mixed slowly to obtain a homogeneous mixture. Throughout the mixing process which took around 1 hour, the temperature of the mixture was maintained at 65°C. Finally, the mixture was allowed to cool to a creamy consistency.

### Protocol used for the evaluation

In this study, a 15X15 cm² cotton fabric swatch was taken. The central circular area corresponding to 33 cm² was applied with 0.5 g of stick base formulation, 0.05 g model sebum and 0.5 mL artificial sweat. Study was done with six replicates. The treated cotton swatches were incubated in a hot air oven at 38°C and 85% RH for 12 h.

Treated cotton swatches were washed in an IFB front loader washing machine, programmed for wash and two rinses. A wash load of approximate 2 ±0.1 kg including polycotton fabrics and the treated swatches was placed into the washing machine. The hardness of water was maintained at 24 FH water (2:1, Ca: Mg). 36 grams of Persil Non-Bio Liquid detergent was added in the dosing chamber at a dosage level of 3gpL. The swatches were dried using tumble dryer (IFB) at hot air mode (45^{°}C) for 90 minutes. The dried swatches were then ironed out according to the nature of the fabric and thus selecting suitable mode settings in the iron box, according to standard consumer practice.

The above process of treating the swatches followed by incubation-washing-drying-ironing the swatches was repeated 4 times and after the final cycle the chromophoric evaluation of the swatches was conducted using a Konika Minolta spectrophotometer Model-2600d. Konika Minolta spectrophotometer was used to measure the stain intensity on the swatches. L*, a*, b* at three different areas of the stain treated swatches and untreated swatches. Following which the Δ b* and Δ E* were calculated subsequently using the ASTM D4265-21 method (Standard guide for Evaluating Stain removal performance in-home laundering). Here the Δ E* and Δ b* were measured as the difference between the fabrics treated with artificial sweat and sebum and the fabrics treated with formulation, artificial sweat, and sebum.

The lower the Δ E* the better is the whiteness and therefore lower Δ E* values indicate better stain removal performance. The higher the Δ b* the higher is the yellowness and therefore lower Δ b* values indicated better stain removal performance.

The above procedure was similarly conducted for polyester swatches and the Δ E* and Δ b* values have been recorded and provided on table 1 below.

### Statistical interpretation using Tukey Kramer HSD

Tukey-Kramer HSD (honestly significant difference) test, is a single-step multiple comparison procedure and statistical test. It can be used to find means that are significantly different from each other and thus comparing more than two population mean.

The standard deviation from the Control was measured based on the mean Δ E* value calculated using 6 fabric swatches (n=6) for each of the formulation in each cycle of the study.

**Table 1:**

| **Ingredients** | Control (wt. % | F1 (wt. %) | F2 (wt. %) | F3 (wt. %) | F4 (wt. %) | F5 (wt. %) | F6 (wt. %) | F7 (wt. %) |
|---|---|---|---|---|---|---|---|---|
| Cyclopentasiloxane | 31.5 | 27.94 | 21.46 | 17.2 | 18.8 | 25 | 24 | 21 |
| Activated Aluminium Zirconium Glycinate | 15 | 15 | 15 | 15 | 15 | 15 | 15 | 15 |
| Stearyl Alcohol | 17 | 17 | 17 | 17 | 17 | 17 | 17 | 17 |
| Hydrogenated Castor Oil (MP80) | 4 | 4 | 4 | 4 | 4 | 4 | 4 | 4 |
| Polyethylene | 0.75 | 0.75 | 0.75 | 0.75 | 0.75 | 0.75 | 0.75 | 0.75 |
| C12-15 Alkyl Benzoate | 15 | 15 | 15 | 15 | 15 | 10 | 10 | 10 |
| PPG-14 Butyl Ether | 11 | 12.56 | 19.04 | 23.3 | 23.7 | 20 | 20 | 20 |
| Silica | 0.75 | 0.75 | 0.75 | 0.75 | 0.75 | 0.75 | 0.75 | 0.75 |
| Steareth-100 | 0.45 | 0.45 | 0.45 | 0.45 | 0.45 | 0.45 | 0.45 | 0.45 |
| PEG-8 Liquid | 2 | 2 | 2 | 2 | 2 | 2 | 2 | 2 |
| PEG-8 Distearate | 0 | 2 | 2 | 2 | 0 | 2 | 2 | 2 |
| Sunflower Seed Oil | 0.5 | 0.5 | 0.5 | 0.5 | 0.5 | 1 | 2 | 5 |
| Dimethicone 50 | 1 | 1 | 1 | 1 | 1 | 1 | 1 | 1 |
| BHT | 0.05 | 0.05 | 0.05 | 0.05 | 0.05 | 0.05 | 0.05 | 0.05 |
| Fragrance | 1 | 1 | 1 | 1 | 1 | 1 | 1 | 1 |
| TOTAL | 100 | 100 | 100 | 100 | 100 | 100 | 100 | 100 |
| Total oil content** | 59 | 57 | 57 | 57 | 59 | 62 | 62 | 62 |
| Weight ratio of PEG-8 distearate to PPG-14 butyl ether | 0 | 0.159 | 0.105 | 0.086 | 0 | 0.1 | 0.1 | 0.1 |

| Stain Removal after 4 cycles | | | | | | | | |
|---|---|---|---|---|---|---|---|---|
| Polyester swatch | | | | | | | | |
| Δ b* | 2.6 | 0.8 | 0.6 | 0.7 | 1.6 | 0.9 | 0.8 | 1.7 |
| Δ E* | 4.6 | 1.8 | 1.3 | 1.5 | 3.3 | 1.2 | 1.0 | 2.5 |
| s.d (Δ E*) | 0.26 | 0.25 | 0.19 | 0.25 | 0.28 | 0.32 | 0.29 | 0.26 |

| Cotton swatch | | | | | | | | |
|---|---|---|---|---|---|---|---|---|
| Δ b* | 4.2 | 1.1 | 1.5 | 1.6 | 2.5 | 1.5 | 1.5 | 2.6 |
| Δ E* | 5.6 | 1.6 | 2.0 | 2.2 | 3.5 | 2.4 | 1.8 | 3.1 |
| s.d (Δ E*) | 0.25 | 0.17 | 0.19 | 0.23 | 0.25 | 0.29 | 0.25 | 0.19 |

| | | | | | | | | |
|---|---|---|---|---|---|---|---|---|
| ** the total oil content includes the sum total of the unsaturated oil, PPG-14 butyl ether, and the preferred oily emollient present in the composition which includes total of the weight of cyclopentasiloxane, C₁₂₋₁₅ Alkyl Benzoate, and dimethicone 50. | | | | | | | | |

The data shows that the composition according to the present invention (F1 to F3) having both a metal based antiperspirant active and unsaturated oil and a weight ratio between PEG 8 distearate to PPG-14 butyl ester within the claimed ranges shows lower Δ E* as it includes the inventive combination of an alkoxylated aliphatic ether and a polyethylene glycol diester which provides improved stain removal and therefore shows better cleaning and improved whiteness. In comparison, the control composition having a metal based antiperspirant active and unsaturated oil but without the PEG-8 distearate shows higher Δ E* and exhibits inferior whiteness and also shows yellowing and the comparative example F4 having metal based antiperspirant active and unsaturated oil and alkoxylated aliphatic ether but no polyethylene glycol diester shows higherΔ E* which indicates that both control and Ex F4 have lower reduction of the stain. The same trend was seen for both the cotton fabric and the polyester fabric.

The examples F2, F5, F6 and F7 shows that the when the content of the unsaturated oil in the composition is varied between 0.5 wt.% and 5 wt.% the inventive combination of the present invention including an alkoxylated aliphatic ether and a polyethylene glycol diester provides improved stain removal. This is demonstrated by the lower Δ E* and lower Δ b* of inventive composition F2, F5, F6 and F7 as compared to the control. The same trend was seen for both the cotton fabric and the polyester fabric.

Example 2: Anti-perspirant stick composition as shown in Table 2 below were prepared in a batch size of 500 to 1000 grams. Initially all the ingredients according to the table 2 were weighed.

To prepare the antiperspirant composition, a beaker was placed in a water bath maintained at a temperature of 85°C. The stearyl alcohol, hydrogenated castor oil and the PEG distearate was added to the beaker and melted. The melted ingredients were then added to a Silverson mixer and mixed slowly to obtain a homogeneous mixture. Throughout the mixing process which took around 1 hour, the temperature of the mixture was maintained at 65°C. Finally, the mixture was allowed to cool to a creamy consistency.

**Table 2**

| **Ingredients** | Control wt.% | Ex A (wt. %) | Ex B (wt. %) | Ex C (wt. %) | Ex D (wt. %) | Ex 1 (wt. %) |
|---|---|---|---|---|---|---|
| Cyclopentasiloxane | 31.5 | 30 | 30 | 17.9 | 17.8 | 35 |
| Activated Aluminium Zirconium Glycinate | 15 | 15 | 15 | 15 | 15 | 15 |
| Stearyl Alcohol | 17 | 17 | 17 | 17 | 17 | 17 |
| Hydrogenated Castor Oil (MP80) | 4 | 4 | 4 | 4 | 4 | 4 |
| Polyethylene | 0.75 | 0.75 | 0.75 | 0.75 | 0.75 | 0.75 |
| C12-15 Alkyl Benzoate | 15 | 15 | 15 | 15 | 15 | 15 |
| PPG-14 Butyl Ether | 11 | 1 | 1 | 20 | 20 | 1 |
| Silica | 0.75 | 0.75 | 0.75 | 0.75 | 0.75 | 0.75 |
| Steareth-100 | 0.45 | 0.45 | 0.45 | 0.45 | 0.45 | 0.45 |
| PEG-8 Liquid | 2 | 2 | 2 | 2 | 2 | 2 |
| PEG-8 Distearate | 0 | 2 | 2 | 0.1 | 0.2 | 2 |
| Sunflower Seed Oil | 0.5 | 10 | 10 | 5 | 5 | 5 |
| Dimethicone 50 | 1 | 1 | 1 | 1 | 1 | 1 |
| BHT | 0.05 | 0.05 | 0.05 | 0.05 | 0.05 | 0.05 |
| Fragrance | 1 | 1 | 1 | 1 | 1 | 1 |
| TOTAL | 100 | 100 | 100 | 100 | 100 | 100 |
| Total oil content** | 59 | 57 | 57 | 58.9 | 58.8 | 57 |
| Weight ratio of PEG-8 distearate to PPG-14 butyl ether | 0 | 2 | 2 | 0.005 | 0.01 | 2 |

| Stain Removal after 4 cycles | | | | | | |
|---|---|---|---|---|---|---|
| Polyester swatch | | | | | | |
| Δ b* | 2.6 | 3 | 2.9 | 4.1 | 4 | 1.8 |
| Δ E* | 4.6 | 5.2 | 5.3 | 6.2 | 6.3 | 2.8 |
| s.d (Δ E*) | 0.26 | 0.26 | 0.26 | 0.19 | 0.25 | 0.6 |
| Cotton swatch | | | | | | |
| Δ b* | 4.2 | 5.1 | 4.8 | 5.8 | 5.8 | 1.9 |
| Δ E* | 5.6 | 6.2 | 5.6 | 6.3 | 6.5 | 2.5 |
| s.d (Δ E*) | 0.25 | 0.23 | 0.17 | 0.25 | 0.36 | 0.6 |
| ** the total oil content includes the sum total of the unsaturated oil, PPG-14 butyl ether, and the | | | | | | |

| | | | | | | |
|---|---|---|---|---|---|---|
| preferred oily emollient present in the composition which includes total of the weight of cyclopentasiloxane, C₁₂₋₁₅ Alkyl Benzoate, and dimethicone 50. | | | | | | |

The data in table 2 shows different comparative examples (Ex A to Ex D) all of which show higher staining and have an inferior stain removal as compared to Control.

Ex A and Ex B with higher than claimed levels of unsaturated oil content shows higher ΔE* value than Control indicating a reduction in the whiteness of the fabrics and also a higher Δ b* compared to Control indicating higher yellowing of the fabrics.

Ex C and Ex D with lower than claimed weight ratio between PEG and PPG showed higher ΔE* and higher Δ b* as compared to the Control indicating a reduction in the whiteness of the fabrics and an increased yellowness of the fabric as compared to Control.

Ex 1 is in accordance to the present invention having ratio between PEG and PPG within the claimed ranges (Ex 1 the weight ratio between PEG-8 Distearate and PPG 14 Butyl ether 2) and shows improved stain removal as compared to Control.

The means of the sample populations were analyzed to envisage statistical significance with 95% confidence interval, i.e. alpha of 0.05 (more than two population means comparison for all pairs using Tukey-Kramer HSD method). According to this analysis it was found that Control and comparative examples (Ex A, Ex B, Ex C and Ex D) generated significantly higher stain on cotton fabric as compared to Ex 1 which was in accordance to the present invention.

## Claims

1. An antiperspirant composition comprising:
i) an alkoxylated aliphatic ether having the general formula (IA) or (IB)
R-O-(C₂H₄O)ₙ-H ........... (IA)
Or,
R-O-(C₃H₆O)ₙ-H .......... (IB);
wherein the C₃H₆ in formulae (IB) is linear or branched, preferably branched;
and where n is an integer from 10 to 18; and, where each R group is independently selected from a straight or a branched linear alkyl group having from 3 to 10 carbon atoms; or an aryl group or a H atom and at least one R group is chosen from the alkyl group, or the aryl group;
ii) a polyalkylene glycol diester of the general formulae (IIA) or (IIB)
R²CO.O-(C₂H₄O)ₙ-CO.R¹ ........................ (IIA)
Or,
R²CO.O-(C₃H₆O)ₙ-CO.R¹ ........................ (IIB)
); wherein the C₃H₆ in formulae (IIB) is linear or branched, preferably branched;
and where n is an integer from 8 to 150; R² and R¹ are each independently selected from the group consisting of C₁₂ to C₁₈ alkyl group or an aryl group.
iii) 0.1 wt.% to 5 wt.% unsaturated oil;
iv) a metal based antiperspirant active;
wherein total oil content including the unsaturated oil present in the composition ranges from 55 wt.% to 65 wt.% and wherein the ratio of the polyethylene glycol diester to alkoxylated aliphatic ether in the composition ranges from 0.05 to 2.

2. A composition according to claim 1 wherein the unsaturated oil has an iodine value of at least 100, preferably from 100 to 150.

3. A composition according to claim 1 or 2 wherein the unsaturated oil is sunflower oil.

4. A composition according to any one of the preceding claims wherein the amount of the metal based antiperspirant active ranges from 1 wt.% to 50 wt.%.

5. A composition according to any one of the preceding claims wherein the weight ratio of the polyethylene glycol diester to alkoxylated aliphatic ether in the composition ranges from 0.09 to 2, more preferably 1 to 2.

6. A composition according to any one of the preceding claims wherein the amount of polyethylene glycol diester present in the composition ranges from 0.1 wt.% to 5 wt.%, preferably 0.1 wt.% to 3 wt.%, still preferably 0.1 wt.% to 2 wt.%.

7. A composition according to any one of the preceding claims wherein the amount of alkoxylated aliphatic ether present in the composition ranges from 5 wt.% to 25 wt.%.

8. A composition according to any one of the preceding claims comprising an oily emollient.

9. A composition according to claim 8 wherein the oily emollient is selected from the group consisting of cyclomethicone, benzoate ester, non-volatile silicone oil preferably dimethicones and mixtures thereof.

10. An antiperspirant composition as claimed in any one of the preceding claims which is delivered as a liquid, lotion, cream, foam, scrub, gel or stick form and may be delivered through a roll-on device or using a propellant containing aerosol can.

11. A method of minimizing staining or yellowish coloration of fabric comprising the steps of (a) applying a composition as claimed in any one of the preceding claims on to a body part, which comes in contact with the fabric when worn by an individual, preferably the axilla followed by (b) washing the fabric.

12. Use of an alkoxylated aliphatic ether with formula (I), a polyethylene glycol diester, 0.1 wt. % to 5 wt. % unsaturated oil, a metal based antiperspirant active in an antiperspirant composition where total oil content including the unsaturated oil in the composition is from 55 wt.% to 65 wt.% according to any one of the preceding claims 1 to 10, for minimizing the staining of the fabrics which come in contact with the composition.

## Patentansprüche

1. Schweißhemmende Zusammensetzung, umfassend:
i) einen alkoxylierten aliphatischen Ether mit der allgemeinen Formel (IA) oder (IB)
R-O-(C₂H₄O)ₙ-H ........................ (IA)
oder
R-O-(C₃H₆O)ₙ-H ............... (IB);
wobei das C₃H₆ in Formel (IB) linear oder verzweigt ist, vorzugsweise verzweigt;
und wobei n eine ganze Zahl von 10 bis 18 ist und wobei jede R-Gruppe unabhängig voneinander aus einer geraden oder einer verzweigten linearen Alkylgruppe mit 3 bis 10 Kohlenstoffatomen oder einer Arylgruppe oder einem H-Atom und mindestens eine R-Gruppe aus der Alkylgruppe oder der Arylgruppe ausgewählt ist;
ii) einen Polyalkylenglycoldiester der allgemeinen Formel (IIA) oder (IIB)
R²CO.O-(C₂H₄O)ₙ-CO.R¹ ..................... (IIA)
oder
R²CO.O-(C₃H₆O)ₙ-CO.R¹ ............ (IIB);
wobei das C₃H₆ in Formel (IIB) linear oder verzweigt ist, vorzugsweise verzweigt;
und wobei n eine ganze Zahl von 8 bis 150 ist; R² und R¹ jeweils unabhängig voneinander aus der Gruppe ausgewählt sind, bestehend aus einer C₁₂- bis C₁₈-Alkylgruppe oder einer Arylgruppe;
iii) 0,1 Gew.-% bis 5 Gew.-% ungesättigtes Öl;
iv) einen schweißhemmenden Wirkstoff auf Metallbasis;
wobei der Gesamtölgehalt, einschließlich des in der Zusammensetzung vorliegenden ungesättigten Öls, in dem Bereich von 55 bis 65 Gew.-% liegt und wobei das Verhältnis des Polyethylenglycoldiesters zum alkoxylierten aliphatischen Ether in der Zusammensetzung in dem Bereich von 0,05 bis 2 liegt.

2. Zusammensetzung nach Anspruch 1, wobei das ungesättigte Öl eine Jodzahl von mindestens 100, vorzugsweise von 100 bis 150, aufweist.

3. Zusammensetzung nach Anspruch 1 oder 2, wobei das ungesättigte Öl Sonnenblumenöl ist.

4. Zusammensetzung nach irgendeinem der vorhergehenden Ansprüche, wobei die Menge des schweißhemmenden Wirkstoffs auf Metallbasis in dem Bereich von 1 Gew.-% bis 50 Gew.-% liegt.

5. Zusammensetzung nach irgendeinem der vorhergehenden Ansprüche, wobei das Gewichtsverhältnis des Polyethylenglycoldiesters zum alkoxylierten aliphatischen Ether in der Zusammensetzung in dem Bereich von 0,09 bis 2, bevorzugter von 1 bis 2, liegt.

6. Zusammensetzung nach irgendeinem der vorhergehenden Ansprüche, wobei die Menge des in der Zusammensetzung vorliegenden Polyethylenglycoldiesters in dem Bereich von 0,1 Gew.-% bis 5 Gew.-%, vorzugsweise 0,1 Gew.-% bis 3 Gew.-%, noch bevorzugter 0,1 Gew.-% bis 2 Gew.-%, beträgt.

7. Zusammensetzung nach irgendeinem der vorhergehenden Ansprüche, wobei die Menge des in der Zusammensetzung vorliegenden alkoxylierten aliphatischen Ethers in dem Bereich von 5 Gew.-% bis 25 Gew.-% liegt.

8. Zusammensetzung nach irgendeinem der vorhergehenden Ansprüche, umfassend einen öligen Weichmacher.

9. Zusammensetzung nach Anspruch 8, wobei der ölige Weichmacher aus der Gruppe ausgewählt ist, bestehend aus Cyclomethicon, Benzoatester, nichtflüchtigem Silikonöl, vorzugsweise Dimethiconen, und Mischungen davon.

10. Schweißhemmende Zusammensetzung, wie in irgendeinem der vorhergehenden Ansprüche beansprucht, die in Form einer Flüssigkeit, Lotion, Creme, eines Schaums, eines Peelings, Gels oder Stifts vorliegt und durch ein Roll-on-Gerät oder unter Verwendung einer Treibmittel enthaltenden Aerosoldose abgegeben werden kann.

11. Verfahren zum Minimieren von Fleckenbildung oder gelblicher Verfärbung von Textilien, umfassend die Schritte (a) Auftragen einer Zusammensetzung, wie in irgendeinem der vorhergehenden Ansprüche beansprucht, auf einen Körperteil, der beim Tragen durch eine Person mit dem Textil in Kontakt kommt, vorzugsweise auf die Achselhöhle, gefolgt von (b) Waschen des Textils.

12. Verwendung eines alkoxylierten aliphatischen Ethers der Formel (I), eines Polyethylenglycoldiesters, 0,1 Gew.-% bis 5 Gew.-% eines ungesättigten Öls, eines schweißhemmenden Wirkstoffs auf Metallbasis in einer schweißhemmenden Zusammensetzung, wobei der Gesamtölgehalt, einschließlich des ungesättigten Öls in der Zusammensetzung, 55 Gew.-% bis 65 Gew.-% beträgt, gemäß irgendeinem der vorhergehenden Ansprüche 1 bis 10, um die Fleckenbildung auf den Textilien, die mit der Zusammensetzung in Kontakt gelangen, zu minimieren.

## Revendications

1. Composition antitranspirante comprenant:
i) un éther aliphatique alcoxylé de formule générale (IA) ou (IB)
R-O-(C₂H₄O)ₙ-H (IA)
ou
R-O-(C₃H₆O)ₙ-H (IB);
dans laquelle le C₃H₆ dans les formules (IB) est linéaire ou ramifié, de préférence ramifié;
et où n est un entier de 10 à 18; et où chaque groupe R est choisi indépendamment parmi un groupe alkyle linéaire ou ramifié ayant de 3 à 10 atomes de carbone; ou un groupe aryle ou un atome H et au moins un groupe R est choisi parmi le groupe alkyle ou le groupe aryle;
ii) un diester de polyalkylène glycol des formules générales (IIA) ou (IIB)
R²CO.O-(C₂H₄O)ₙ-CO.R¹ (IIA)
ou
R²CO.O-(C₃H₆O)ₙ-CO.R¹ (IIB);
dans laquelle le C₃H₆ dans les formules (IIB) est linéaire ou ramifié, de préférence ramifié;
et où n est un entier de 8 à 150; R² et R¹ sont choisis chacun indépendamment dans le groupe consistant en un groupe alkyle en C₁₂ à C₁₈ ou un groupe aryle,
iii) 0,1 % en poids à 5 % en poids d'huile insaturée;
iv) un actif antitranspirant à base de métal;
dans laquelle la teneur totale en huile, y compris l'huile insaturée présente dans la composition, est de 55 % en poids à 65 % en poids et dans laquelle le rapport du diester de polyéthylène glycol à l'éther aliphatique alcoxylé dans la composition est de 0,05 à 2.

2. Composition selon la revendication 1, dans laquelle l'huile insaturée a un indice d'iode d'au moins 100, de préférence de 100 à 150.

3. Composition selon la revendication 1 ou 2, dans laquelle l'huile insaturée est l'huile de tournesol.

4. Composition selon l'une quelconque des revendications précédentes, dans laquelle la quantité d'actif antitranspirant à base de métal est de 1 % en poids à 50 % en poids.

5. Composition selon l'une quelconque des revendications précédentes, dans laquelle le rapport pondéral du diester de polyéthylène glycol à l'éther aliphatique alcoxylé dans la composition est de 0,09 à 2, de préférence de 1 à 2.

6. Composition selon l'une quelconque des revendications précédentes, dans laquelle la quantité de diester de polyéthylène glycol présente dans la composition est de 0,1 % à 5 % en poids, de préférence de 0,1 % à 3 % en poids, de préférence encore de 0,1 % à 2 % en poids.

7. Composition selon l'une quelconque des revendications précédentes, dans laquelle la quantité d'éther aliphatique alcoxylé présente dans la composition est de 5 % à 25 % en poids.

8. Composition selon l'une quelconque des revendications précédentes, comprenant un émollient huileux.

9. Composition selon la revendication 8, dans laquelle l'émollient huileux est choisi dans le groupe consistant en la cyclométhicone, un ester benzoate, une huile de silicone non volatile, de préférence les diméthicones et leurs mélanges.

10. Composition antitranspirante selon l'une quelconque des revendications précédentes qui est délivrée sous forme de liquide, de lotion, de crème, de mousse, de gommage, de gel ou de bâton, et peut être délivrée au moyen d'un dispositif à bille ou à l'aide d'une bombe aérosol contenant un propulseur.

11. Procédé pour minimiser les taches ou la coloration jaunâtre d'un tissu comprenant les étapes de:
(a) application d'une composition selon l'une quelconque des revendications précédentes sur une partie du corps qui vient en contact avec le tissu lorsqu'il est porté par un individu, de préférence l'aisselle, puis (b) lavage du tissu.

12. Utilisation d'un éther aliphatique alcoxylé de formule (I), d'un diester de polyéthylène glycol, de 0,1 % en poids à 5 % en poids d'huile insaturée, d'un actif antitranspirant à base de métal dans une composition antitranspirante où la teneur totale en huile, y compris l'huile insaturée, dans la composition est de 55 % en poids à 65 % en poids selon l'une quelconque des revendications 1 à 10 précédentes, pour minimiser les taches des tissus qui viennent en contact avec la composition.
